# EUROPEAN PATENT SPECIFICATION

(11) **EP 1 342 794 B1**
(45) Date of publication and mention of the grant of the patent: **14.12.2005**
(21) Application number: 02004954.0
(22) Date of filing: 05.03.2002
(51) Int. Cl.: C12Q 1/68

(54) **Method and device for determination of tissue specificity of free floating DNA in bodily fluids**
Verfahren und Vorrichtung zur Bestimmung der Gewebespezifität von freier DNA in Körperflüssigkeiten
Procédé et dispositif pour la détérmination de la spécifité au tissus et d'ADN libre dans fluids corporels

(43) Date of publication of application: 10.09.2003
(73) Proprietor: Epigenomics AG, 10435 Berlin (DE)
(72) Inventor: Berlin, Kurt, 14532 Stahnsdorf (DE); Sledziewski, Andrzej, Shoreline, WA 98117 (US)
(74) Representative: Krauss, Jan

(56) References cited:
- WO-A-96/40995
- WO-A-98/08980
- WO-A-98/56952
- US-A- 5 496 699
- US-B1- 6 214 556
- US-B1- 6 331 393
- USADEL HENNING ET AL: "Quantitative adenomatous polyposis coli promoter methylation analysis in tumor tissue, serum, and plasma DNA of patients with lung cancer." CANCER RESEARCH, vol. 62, no. 2, 15 January 2002 (2002-01-15), pages 371-375, XP001080048 January 15, 2002 ISSN: 0008-5472
- SHAPIRO B ET AL: "DETERMINATION OF CIRCULATING DNA LEVELS IN PATIENTS WITH BENIGN OR MALIGNANT GASTRO INTESTINAL DISEASE" CANCER (PHILADELPHIA), vol. 51, no. 11, 1983, pages 2116-2120, XP001083835 ISSN: 0008-543X
- LEON S A ET AL: "FREE DNA IN THE SERUM OF CANCER PATIENTS AND THE EFFECT OF THERAPY" CANCER RESEARCH, vol. 37, no. 3, 1977, pages 646-650, XP001083834 ISSN: 0008-5472
- GIACONA M B ET AL: "CELL-FREE DNA IN HUMAN BLOOD PLASMA: LENGTH MEASUREMENTS IN PATIENTS WITH PANCREATIC CANCER AND HEALTHY CONTROLS" PANCREAS, RAVEN PRESS, NEW YORK, NY, US, vol. 17, no. 1, July 1998 (1998-07), pages 89-97, XP000992791 ISSN: 0885-3177
- SORENSON G D ET AL: "SOLUBLE NORMAL AND MUTATED DNA SEQUENCES FROM SINGLE-COPY GENES IN HUMAN BLOOD" CANCER EPIDEMIOLOGY, BIOMARKERS AND PREVENTION, AMERICAN ASSOCIATION FOR CANCER RESEARCH,, US, vol. 3, January 1994 (1994-01), pages 67-71, XP001064067 ISSN: 1055-9965
- BOTEZATU IRINA ET AL: "Genetic analysis of DNA excreted in urine: A new approach for detecting specific genomic DNA sequences from cells dying in an organism." CLINICAL CHEMISTRY, vol. 46, no. 8 Part 1, August 2000 (2000-08), pages 1078-1084, XP001080049 ISSN: 0009-9147

## Description

### BACKGROUND OF THE INVENTION

This invention relates to methods for detecting free floating nucleic acids, as present in not cellular bound nucleic acids in bodily fluids like plasma or serum fractions of human or animal blood or in any other tissue samples derived from the human or animal body in order to diagnose a cell proliferative disease. Specifically the invention relates to the detection of increased levels of nucleic acids in bodily fluids. Furthermore the invention allows to determine the source of the enriched DNA by measuring the ratio of DNA originating from a certain organ versus total DNA from other organs in a given bodily fluid sample by specifying the DNA's methylation pattern. This can be done with or without increasing the DNA concentration of a given biological sample. In a preferred embodiment a further analysis of this methylation pattern allows for the detection of the presence of tumorous or otherwise proliferative disease in said organ.

### PRIOR ART

### DNA based assays to detect cancer

A number of genetic alterations like mutations in certain genes, but also loss of heterozygosity and microsatellite instability at certain loci can be detected in DNA samples from tumor tissue. These DNA alterations can be detected in DNA retrieved from the tumor tissue of a patient. In some cases it has been reported that these alterations were also found in DNA samples from serum or blood or the sputum of those tumor patients.
It is known that cigarette smokers have increased bronchial secretions that contain exfoliated cells from the bronchial tree. From analyzing these excreted cells, premalignant cytological changes could be detected several years before a clinical diagnosis of lung cancer in high risk patients (Saccomanno et al. (1974) Cancer (Phila.), 33: 256-270). These studies were not easily reproducible and required specific skills in the person which analyzed those samples. Therefore, to enhance the predictive value of the sputum samples, it has been suggested to use molecular assays, for example to detect mutations within the K-ras gene or microsatellite alterations specific to the tumor (Mao et al. (1994) Proc. Natl. Acad. Sci. USA, 91: 9871-9875 and Mao et al. (1994) Cancer Res., 54: 1634-1637). K-ras as well as p53 mutations have been detected in bodily fluids as found in cytological samples of the sputum and bronchial lavage of lung cancer patients and chronic smokers (Kersting et al. (2000) J. Clin. Oncol., 18: 3221-3229 and Ahrendt et al. (1999) J. Natl. Cancer Inst. (Bethesda), 91: 332-339). Knowing the nucleic acid sequences of specific marker genes involved in a certain type of cancer like, for example, lung cancer enabled the analysis of these sputum samples and allowed to predict the development of lung cancer in high risk patients. More relevant information on this matter can be found in patent WO 95/16792 by Maurice Stroun, Philippe Anker and Valeri Vasioukhin. However, these methods are not ideal as they lack sensitivity and the overall prevalence of these changes in non-small cell lung cancer is less than 25% (Palmisano et al. (2000), Cancer Res. 60: 5954-5958). Also for prostate cancer it has been reported that the inactivation of the HPC2/ELAC2 gene via LOH is a relatively uncommon event (Wu et al. (2001) Cancer Res 61: 8651-8653). Another factor highly correlated with the occurrence of tumors is the hypermethylation of certain promoters and promoter regions.

### Methylation

In recent decades in molecular biology studies have focussed primarily on genes, the translation of those genes into RNA, and the transcription of the RNA into protein. There has been a more limited analysis of the regulatory mechanisms associated with gene control. Gene regulation, for example, at what stage of development of the individual a gene is activated or inhibited, and the tissue specific nature of this regulation is less understood. However, it can be correlated with a high degree of probability to the extent and nature of methylation of the gene or genome. From this observation it is reasonable to infer that pathogenic genetic disorders may be detected from irregular genetic methylation patterns and this has been shown for a number of cases. In addition this invention discloses a method on how to determine the origin of DNA in a bodily fluid by analyzing its methylation pattern in order to detect aberrant levels of DNA deriving from a certain organ, indicating a cell proliferative disease of said organ.
In higher order eukaryotes DNA is methylated nearly exclusively at cytosines located 5' to guanosine in the CpG dinucleotide. This modification has important regulatory effects on gene expression, especially when involving CpG rich areas, known as CpG islands, located in the promoter regions of many genes. While almost all gene-associated islands are protected from methylation on autosomal chromosomes, extensive methylation of CpG islands has been associated with transcriptional inactivation of selected imprinted genes and genes on the inactive X-chromosome of females. Aberrant methylation of normally unmethylated CpG islands has been described as a frequent event in immortalized and transformed cells, and has been associated with transcriptional inactivation of defined tumor suppressor genes in human cancers. Human cancer cells typically contain somatically altered genomes, characterized by mutation, amplification, or deletion of critical genes. In addition, the DNA template from human cancer cells often displays somatic changes in DNA methylation (E. R. Fearon, et al., Cell, 61:759, 1990; P. A. Jones, et al., Cancer Res., 46: 461, 1986; R. Holliday, Science, 238: 163, 1987; A. De Bustros, et al., Proc. Natl. Acad. Sci., USA, 85: 5693, 1988; P. A. Jones, et al., Adv. Cancer Res., 54:1, 1990; S. B. Baylin, et al., Cancer Cells, 3 :383, 1991; M. Makos, et al., Proc. Natl. Acad. Sci., USA, 89: 1929, 1992; N. Ohtani-Fujita, et al., Oncogene, 8:1063, 1993). However, the precise role of abnormal DNA methylation in human tumorigenesis has not been established. DNA methylases transfer methyl groups from the universal methyl donor S-adenosyl methionine to specific sites on the DNA.
Several biological functions have been attributed to the methylated bases in DNA. The most established biological function is the protection of the DNA from digestion by cognate restriction enzymes. The restriction modification phenomenon has, so far, been observed only in bacteria. Mammalian cells, however, possess a different methylase that exclusively methylates cytosine residues on the DNA, that are 5' neighbors of guanine (CpG). This methylation has been shown by several lines of evidence to play a role in gene activity, cell differentiation, tumorigenesis, X-chromosome inactivation, genomic imprinting and other major biological processes (Razin, A., H., and Riggs, R. D. eds. in DNA Methylation Biochemistry and Biological Significance, Springer-Verlag, N.Y., 1984).

Although the exact mechanisms by which DNA methylation effects DNA transcription are unknown, the relationship between disease and methylation has been well documented. Misregulation of genes may be predicted by comparing their methylation pattern with phenotypically 'normal' expression patterns. The following are cases of disease associated with modified methylation patterns, the specific role of methylation in cancer is described in the next paragraph:
- Hodgkin's disease (Garcia JF et al "Loss of p 16 protein expression associated with methylation of the p16INK4A gene is a frequent finding in Hodgkin's disease" Lab invest 1999 Dec; 79 (12): 1453-9)
- Prader-Willi/Angelman's syndrome (Zeschnigh et al "Imprinted segments in the human genome: different DNA methylation patterns in the Prader Willi/Angelman syndrome region as determined by the genomic sequencing method" Human Mol. Genetics (1997) (6) 3 pp 387-395)
- ICF syndrome (Tuck-Muller et al "CMDNA hypomethylation and unusual chromosome instability in cell lines from ICF syndrome patients" Cytogenet Call Genet 2000; 89(1-2):121-8
- Dermatofibroma (Chen TC et al "Dermatofibroma is a clonal proliferative disease" J Cutan Pathol 2000 Jan;27 (1):36-9)
- Hypertension (Lee SD et al. " Monoclonal endothelial cell proliferation is present in primary but not secondary pulmonary hypertension" J clin Invest 1998 Mar 1, 101 (5):927-34)
- Autism (Klauck SM et al. "Molecular genetic analysis of the FMR-1 gene in a large collection of autistic patients" Human Genet 1997 Aug; 100 (2) : 224-9)
- Fragile X Syndrome (Hornstra IK et al. " High resolution methylation analysis of the FMR1 gene trinucleotide repeat region in fragile X syndrome" Hum Mol Genet 1993 Oct, 2(10):1659-65)
- Huntigton's disease (Ferluga J et al. "possible organ and age related epigenetic factors in Huntington's disease and colorectal carcinoma" Med hypotheses 1989 May;29(1);51-4

All of documents cited herein are hereby incorporated by reference.

### Hypermethylation and cancer

DNA methylation can down-regulate gene expression, and when it does so inappropriately it might lead to a shutting off of tumor suppressor genes, for instance and cause cancer. Consequently, it has been shown frequently that certain regions of the genome are hypermethylated in tumor tissue when this is not the case in neighboring unaffected cells. A well investigated system is the inactivation of GSTP1 (glutathione-S-transferase promoter 1) by CpG island hypermethylation, the most common somatic genome alteration yet reported for human prostate cancer, occurs early during human prostatic carcinogenesis and results in a loss of GSTP1 caretaker function, leaving prostate cells with inadequate defenses against oxidant and electrophile carcinogens. The genetic diagnosis of prostate cancer via detection of the methylation status of the GSTP1 has been described in the patent US 5,552,277. Another example out of many more is described in the following paper: Yanagisawa Y et al. (2000) "Methylation of the hMLHI promoter in familial gastric cancer with microsatellite instability" Int J Cancer 85:50-3).
A recent example for the correlation between hypermethylation and cancer is given by Maruyama et al. when they reported a positive correlation between the median methylation index of a number of selected genes and the prognosis of bladder cancer development in December 2001 (Maruyama et al. (2001) Cancer Res 61: 8659-8663). Methylation of CDH1, FHIT, and a high MI were associated with shortened survival. CDH1 methylation positive status was independently associated with poor survival in multivariate analyses. The authors conclude that the methylation profile may be a potential new biomarker of risk prediction in bladder cancer, but as they only analysed biopsy samples this would require surgical operation on a patient. However more recent studies have highlighted the possibility to detect DNA methylation in DNA from bodily fluids rather than from tumor tissue itself.

### DNA methylation in bodily fluids

For example in DNA from exfoliated cells in sputum samples from lung cancer patients or high risk patients the p16 tumor suppressor gene promoter and/or 06-methylguanine-DNA methyltransferase promoters could be shown to be aberrantly methylated. The aberrant methylation could be detected in DNA from sputum in 100% of patients with squamous cell lung carcinoma up to 3 years before clinical diagnosis (Palmisano et al. (2000), Cancer Res. 60: 5954-5958).
When the methylation status of the p15 and p16 promoter region from tumor DNA and blood (plasma, serum and buffy coat samples) DNA from hepatocellular carcinoma patients was investigated, 87% of the patients with tumor methylation also showed methylated DNA in the blood stream. None of the control samples were methylation positive (Wong et al. (2000) Clin Cancer Res 6(9):3516-3521). In addition a study on Head and Neck cancer revealed a correlation between serum DNA methylation and tumor DNA methylation of 42% (Sanchez-Cespedes et al. (2000) Cancer Res 60: 892-895).
Methylated DNA as a tumor marker is not only restricted to the sputum or blood stream, but can - at least in prostate carcinoma patients - also be found in urine or ejaculate samples. In this study 94% of the tumor DNA samples were methylated, 72% of the plasma or serum samples, 50% of ejaculate samples and 36% of urine samples (after prostatic massage in order to release prostatic secretions) from patients with prostate cancer whereas no methylation was detected in samples from the control group (Cairns et al. (2001) Clin Cancer Res 7: 2727-2730).

The detection of aberrant promoter region methylation constitutes a promising approach for using DNA methylation based marker assays for the early detection of common human cancers. As hypermethylation is involved in a wide range of cancer types one can think of a number of similar approaches for other types of cancer. For an overview see:
Esteller, M., Corn, P. G., Baylin, S. B., Herman, J. G. (2001). A Gene Hypermethylation Profile of Human Cancer. Cancer Res 61: 3225-3229
or for a selection of recent publications on the matter:
Byun, D.-S., Lee, M.-G., Chae, K.-S., Ryu, B.-G., Chi, S.-G. (2001). Frequent Epigenetic Inactivation of RASSF1A by Aberrant Promoter Hypermethylation in Human Gastric Adenocarcinoma. Cancer Res 61: 7034-7038.
Agathanggelou A., Honorio S., Macartney D. P., Martinez A., Dallol A., Rader J., Fullwood P., Chauhan A., Walker R., Shaw J. A., Hosoe S., Lerman M. I., Minna J. D., Maher E. R., Latif F. (2001). Methylation associated inactivation of RASSF1A from region 3p21.3 in lung, breast and ovarian tumours. Oncogene, 20: 1509-1518.
Dong, S. M., Kim, H.-S., Rha, S.-H., Sidransky, D. (2001). Promoter Hypermethylation of Multiple Genes in Carcinoma of the Uterine Cervix. Clin Cancer Res 7: 1982-1986.
Herman J. G., Latif F., Weng Y. K., Lerman M. I., Zbar B., Liu S., et al (1994) Silencing of the VHL tumor suppressor gene by DNA methylation in renal carcinomas. Proc. Natl. Acad. Sci. USA, 91: 9700-9704. Usadel, H. et al. (2002). Quantitative adenomatous polyposis coli promoter methylation analysis in tumor tissue, serum, and plasma of patients with lung cancer.

### Methods to detect methylated DNA

In the previous paragraphs the significance of methylation of certain cytosine bases for gene activity, cell differentiation, tumorigenesis, X-chromosome inactivation, genomic imprinting and other major biological processes (Razin, A., H., and Riggs, R. D. eds. in DNA Methylation Biochemistry and Biological Significance, Springer-Verlag, N.Y., 1984) has been described. The cytosin's modification in form of methylation contains significant information. It is obvious that the identification of 5-methylcytosine in a DNA sequence as opposed to unmethylated cytosine is of greatest importance to analyze its role further. But, because the 5-Methylcytosine behaves just as a cytosine for what concerns its hybridization preference (a property relied on for sequence analysis) its positions can not be identified by a normal sequencing reaction.
Furthermore, in a PCR amplification this relevant epigenetic information, methylated cytosine or unmethylated cytosine, will be lost completely.
Several methods are known that solve this problem. Usually the genomic DNA is treated with a chemical or enzyme leading to a conversion of the cytosine bases, which consequently allows to differentiate the bases afterwards. Some restriction enzymes are capable of differentiating between methylated and unmethylated DNA.
A relatively new and currently the most frequently used method for analyzing DNA for 5-methylcytosine is based upon the specific reaction of bisulfite with cytosine which, upon subsequent alkaline hydrolysis, is converted to uracil, whereas 5-methylcytosine remains unmodified under these conditions (Shapiro et al. (1970) Nature 227: 1047). Uracil corresponds to thymidine in its base pairing behavior, whereas 5-methylcytosine doesn't change its chemical properties under this treatment and corresponds to guanin. Consequently, the original DNA is converted in such a manner that methylcytosine, which originally could not be distinguished from cytosine by its hybridization behavior, can now be detected as the only remaining cytosine using "normal" molecular biological techniques, for example, by amplification and hybridization or sequencing. All of these techniques are based on base pairing which can now be fully exploited.

Comparing the sequences of the DNA with and without bisulfite treatment allows an easy identification of those bases that have been methylated.
An overview of the further known methods of detecting 5-methylcytosine may be gathered from the following review article: Rein, T., DePamphilis, M. L., Zorbas, H., Nucleic Acids Res. 1998, 26, 2255.
In terms of sensitivity, the prior art is defined by a method, which encloses the DNA to be analyzed in an agarose matrix, thus preventing the diffusion and renaturation of the DNA (bisulfite reacts with single-stranded DNA only), and which replaces all precipitation and purification steps with fast dialysis (Olek A, Oswald J, Walter J. (1996) A modified and improved method for bisulphite based cytosine methylation analysis. Nucleic Acids Res. 24: 5064-6). Using this method, it is possible to analyze individual cells, which illustrates the potential of the method.

To date, barring few exceptions (e.g., Zeschnigk M, Lich C, Buiting K, Doerfler W, Horsthemke B. (1997) A single-tube PCR test for the diagnosis of Angelman and Prader-Willi syndrome based on allelic methylation differences at the SNRPN locus. Eur J Hum Genet. 5: 94-8) the bisulfite technique is only used in research. Always, however, short, specific fragments of a known gene are amplified subsequent to a bisulfite treatment and either completely sequenced (Olek A, Walter J. (1997) The pre-implantation ontogeny of the H19 methylation imprint. Nat Genet. 3: 275-6) or individual cytosine positions are detected by a primer extension reaction (Gonzalgo ML and Jones PA. (1997) Rapid quantitation of methylation differences at specific sites using methylation-sensitive single nucleotide primer extension (Ms-SNuPE). Nucleic Acids Res. 25 :2529-31, WO 95/00669) or by enzymatic digestion (Xiong Z, Laird PW. (1997) COBRA: a sensitive and quantitative DNA methylation assay. Nucleic Acids Res. 25: 2532-4).

Another technique to detect hypermethylation is the so-called methylation specific PCR (MSP) (Herman JG, Graff JR, Myohanen S, Nelkin BD and Baylin SB. (1996), Methylation-specific PCR: a novel PCR assay for methylation status of CpG islands. Proc Natl Acad Sci U S A. 93: 9821-6). The technique is based on the use of primers that differentiate between a methylated and a non-methylated sequence if applied after bisulfite treatment of said DNA sequence. The primer either contains a guanine at the position corresponding to the cytosine in which case it will after bisulfite treatment only bind if the position was methylated. Or the primer contains an adenine at the corresponding cytosine position and therefore only binds to said DNA sequence after bisulfite treatment if the cytosine was unmethylated and has hence been altered by the bisulfite treatment so that it hybridizes to adenine. With the use of these primers, amplicons can be produced specifically depending on the methylation status of a certain cytosine and will as such indicate its methylation state.
Another new technique is the detection of methylation via Taqman PCR, also known as MethylLight (WO 00/70090). With this technique it became feasible to determine the methylation state of single or of several positions directly during PCR, without having to analyze the PCR products in an additional step.

In addition, detection by hybridization has also been described (Olek et al., WO 99/28498). Further publications dealing with the use of the bisulfite technique for methylation detection in individual genes are: Grigg G, Clark S. (1994) Sequencing 5-methylcytosine residues in genomic DNA. Bioessays 16: 431-6; Zeschnigk M, Schmitz B, Dittrich B, Buiting K, Horsthemke B, Doerfler W. (1997) Imprinted segments in the human genome: different DNA methylation patterns in the Prader-Willi/Angelman syndrome region as determined by the genomic sequencing method. Hum Mol Genet. 6: 387-95; Feil R, Charlton J, Bird AP, Walter J, Reik W. (1994) Methylation analysis on individual chromosomes: improved protocol for bisulphite genomic sequencing. Nucleic Acids Res. 22: 695-6; Martin V, Ribieras S, Song-Wang X, Rio MC, Dante R. (1995) Genomic sequencing indicates a correlation between DNA hypomethylation in the 5' region of the pS2 gene and its expression in human breast cancer cell lines. Gene 157 : 261-4; WO 97/46705, WO 95/15373 and WO 97/45560.

### Elevated Levels of Circulating DNA

Another characteristic property of cancer and other cell proliferative diseases is an increased amount of free floating, circulating DNA in blood and/or serum. Also cell death caused by for example toxic doses of bacterial lipopolysaccharide, HgCl2, CCl4, cyclophosphamide and hydroxyurea triggers the release of products of chromatin catabolism, particularly of DNA into extracellular spaces. At least those have been shown to be responsible for the release of extracellular DNA in plasma in mice, in a dose dependent relationship. Hence it was suggested to use the quantitation of extracellular DNA for investigating *in vivo* cell death phenomena induced by toxic agents and drugs (Bret et al. (1990) Toxicology 61(3): 283-92).
It is known that plasma DNA content reflects the amount of cell death occurring in the whole body and is increased during destructive pathological processes, including cancer. Increased DNA contents in serum have been found in correlation with Systemic Lupus Erythematosus (Leon et al. (1977) Cancer Res. 37: 646-650), malignant gastrointestinal disease (Shapiro et al. (1983), Cancer 51:2116-2120), pancreatic cancer (Anker et al. (1999), Cancer Metastasis Rev. 18: 65-73) and lung cancer (Maebo A. (1990), Jap J Thoraic Dis 28: 1085-1091 and Fournié et al. (1995), Cancer Let 2: 221-227). Whilst healthy human beings have free floating DNA levels in the range of 2-30 ng/ml, cancer patients, more specifically patients with Systemic Lupus Erythematosus in an early study from 1977 showed levels of up to 180 ng/ml. (Leon et al. (1977) Cancer Res. 37: 646-650). In a publication from Jahr et al. a table is shown which describes plasma DNA levels from 23 patients grouped into 12 different tumor groups. In the most extreme case the DNA level was increased 100x compared to the mean value of DNA level in healthy patients. They concluded that elevated levels of circulating DNA appear to be a characteristic feature of most, but not all of the carcinoma diseases. The determined level of circulating DNA alone could not be correlated to the type of cancer or to the clinical status. But it has to be said that in Jahrs study no more than 4 repeats of any one tumor have been performed (Jahr et al. (2001), Cancer Res 61: 1659-1655).
Jahr et al. tried to analyze how much of this circulating DNA originates from tumor cells. It was reported from studies based on tumor specific microsatellite changes that nearly all the circulating plasma DNA originated from tumor cells (Goessl C. et al. (1998) Cancer Res., 58: 4728-4732). Other studies contrarily detected wild type DNA in the plasma of nearly all of the cancer patients. To be able to distinguish between tumor DNA and non tumor DNA they determined the DNA's methylation status, assuming that methylated DNA derived from the tumor tissue only and non methylated DNA from healthy cells. It was found that when the DNA count in the plasma was very high, the percentage of methylation was quite low, whereas when the DNA level was rather low the percentage of methylated DNA was - in one case at least - up to above 90%. The authors stress the fact that it would be difficult to investigate whether the unmethylated DNA originates from the neighboring tumor tissue or from some other source "because DNA markers that distinguish defined cell types are not available". In this paper evidence is discussed which supports the idea that the circulating DNA origins from apoptotic and necrotic cells.
Although the exact mechanism of the release of circulating DNA remains to be proved, an active release of circulating DNA from highly proliferating cells has also been proposed (Anker et al. (1999), Cancer Metastasis Rev. 18: 65-73). Herein the authors discuss why the origin of circulating DNA in the blood stream of cancer patients is most likely to be 'active release', rather than lysis of circulating cancer cells, necrosis or apoptosis.
Botezatu et al. described how to detect extracellular DNA in the urine and how to analyze this DNA in order to diagnose cancer (Botezatu et al. (2000) Genetic analysis of DNA excreted in urine: a new approach for detecting specific genomic DNA sequences from dying cells in an organism. Clin Chem 46:1078-1084). Unlike previous work illustrating the diagnostic use of urine for cancer detection (Mao L. (1996) Genetic alterations as clonal markers for bladder cancer detection in urine. J Cell Biochem Suppl 25:191-196 and Eisenberger et al. (1999) Diagnosis of renal cancer by molecular urinalysis. J Natl Cancer Inst 91: 2028-2032), the cancer types chosen by Botezatu et al., namely pancreatic and colorectal carcinomas, are not of urologic origin. Previous work has indicated that pancreatic and colorectal cancer cells (Anker et al. (1997) K-ras mutations are found in DNA extracted from the plasma of patients with colorectal cancer. Gastroenterology 112: 1114-1120) can release tumoral DNA into the plasma. The new results by Botezatu et al. go one step further by suggesting that tumor DNA, following its release into the blood stream, will be excreted into the urine in sizes large enough for PCR analysis and hence applicable to our techniques on how to determine methylation patterns.
The data by Botezatu et al. include only patients with relatively advanced diseases (stages III and IV), the applicability of urine DNA analysis to the detection of early nonurologic malignancies remains to be demonstrated in future studies (Lo et al. (2000) Molecular Testing of Urine: Catching DNA on the Way Out. Clinical Chemistry 46: 1039-1040).

### Methods on quantitation of nucleic acids

Accurately determining the DNA concentrations of crude chromosomal or purified plasmid DNA samples is an essential step in quantitative manipulations of DNA. Two types of methods are widely used to measure the amount of nucleic acid in a preparation. If the sample is pure (i.e. without significant amounts of contaminants such as proteins, phenol, agarose or other nucleic acids), spectrophotometric measurement of the amount of ultraviolet irradiation absorbed by the bases is simple and accurate. Two different techniques rely on spectrophotometric and/or fluorometric analyses, for example to determine the concentration of a dilute sample of plasmid DNA purified by two passes through an ethidium bromide - cesium chloride (EtBr-CsCl) centrifugation gradient. The sample can either be tested on an for example LKB Biochrom Ultrospec II spectrophotometer for absorbance at wavelengths of 260 nm and 280 nm, or it can be tested for emission of 460 nm on the Hoefer TKO 100 mini-fluorometer in the presence of bisbenzimidazole, a fluorescent dye known as Hoechst H 33258 (manufactured by American Hoechst Corporation), that has an excitation maximum at 356 nm and an emission maximum of 458 when bound to DNA (Labarca and Paigen (1980) Anal. Biochem. 102, 344-352). The spectrophotometer detects absorbance due to RNA as well as DNA, while the Hoechst dye used in the fluorometer interacts specifically with adenosine and thymidine residues of DNA. Due to the highly specific nature of the Hoechst dye the mini-fluorometer seems to be most accurate for quantitation of crude chromosomal DNA, but less reliable for plasmids and other DNA of limited complexity.
If the amount of DNA or RNA is very small or if the sample contains significant quantities of impurities, the amount of nucleic acid can be estimated from the intensity of fluorescence emitted by ethidium bromide molecules intercalated into the DNA (Sambrook; Fritsch and Maniatis (1989) Molecular Cloning - A laboratory manual (second edition) 3: E.5). A simple application of this general approach is the use of EtBr agarose plates. DNA samples of 2-10 ul are spotted onto 1% agarose containing 0.5 ug/ml EtBr within a Petri dish. Afterwards, the plate is exposed to UV light and photographed. Another variation is to mix 5-10 ul of a 0.5 ug/ml solution of EtBr with 10 ul of DNA spotted onto plastic film wrap or a siliconized glass slide placed on top of a UV transilluminator. The advantage of this method is that DNA samples with as little as 1-10 ng of DNA can be quantitated within minutes. The disadvantage is the intercalation of the dye with RNA as well as DNA and its limitation to double stranded DNA.
Other methods for quantitating DNA are for example, Invitrogen's nucleic acid quantitation DNA Dipstick [TM] kit, which is claimed to be sensitive enough to detect as little as 0.1 ng/ul of nucleic acid. Unfortunately, the method cannot be used with samples containing more than 10 ng/ul of nucleic acids. (see: Trends in Biochemical Sciences 19, 46-47).

### Methods on detection of specific DNA

Methods to detect and quantify specific nucleic acids are used in detecting microorganisms, viruses and biological molecules. Hence they are used in human and veterinary medicine, food processing and environmental testing. Additionally, the detection and/or quantification of specific biomolecules from biological samples (e.g. tissue, sputum, urine, blood, semen, saliva) has applications in forensic science, such as the identification and exclusion of criminal suspects and paternity testing as well as medical diagnostics. However the majority of such methods is based on 2 techniques: hybridization and PCR. Both of which detect and quantify a certain specific part of the genomic DNA.
Hybridization is known as one of the methods to detect a nucleic acid having a specified base sequence (hereafter referred to as "target nucleic acid"). This method employs an oligonucleotide probe having a base sequence capable of hybridizing to the target nucleic acid as a detection probe to form a hybrid, and performs detection of the target nucleic acid by detecting the hybrid through various detection means.
In patent US 6,228,592 the drawbacks of this technique get mentioned, especially when trying to apply those to detecting a specific sequence in a surrounding environment like a biologically active fluid or especially in a living cell. When a detection probe is introduced into the cytoplasm, it will a) rapidly move to the nucleus and b) the probe or the hybrid between the detection probe and the target nucleic acid is rapidly digested by various kinds of nuclease existing in the cytoplasm, which renders the detection of the target nucleic acid difficult. This can be circumvented by using an oligonucleotide probe having a base sequence capable of hybridizing to the specified base sequence of a target nucleic acid, which is bound to a nuclear membrane unpermeable molecule via a linker and labeled with a fluorescent dye; forming a hybrid between the target nucleic acid and the probe. A change in fluorescence of the fluorescent dye due to formation of the hybrid thereby detects the existence of the target nucleic acid in the cytoplasm of a living cell or any other background contaminated with DNAses.
Another type of process for the detection of hybridized nucleic acid takes advantage of the polymerase chain reaction (PCR). The PCR process is well known in the art (U.S. Pat. Nos. 4,683,195, 4,683,202, and 4,800,159). To briefly summarize PCR, nucleic acid primers, complementary to opposite strands of a nucleic acid amplification target sequence, are permitted to anneal to the denatured sample. A DNA polymerase (typically heat stable) extends the DNA duplex from the hybridized primer. The process is repeated to amplify the nucleic acid target. If the nucleic acid primers do not hybridize to the sample, then there is no corresponding amplified PCR product. In this case, the PCR primer acts as a hybridization probe. PCR-based methods are of limited use for the detection of nucleic acid of unknown sequence.
In a PCR method, the amplified nucleic acid product may be detected in a number of ways, e.g. incorporation of a labeled nucleotide into the amplified strand by using labeled primers. Primers used in PCR have been labeled with radioactivity, fluorescent dyes, digoxygenin, horseradish peroxidase, alkaline phosphatase, acridinium esters, biotin and jack bean urease. PCR products made with unlabeled primers may be detected in other ways, such as electrophoretic gel separation followed by dye-based visualization.
Fluorescence techniques are also known for the detection of nucleic acid hybrids. U.S. Pat. No. 5,691,146 describes the use of fluorescent hybridization probes that are fluorescence-quenched unless they are hybridized to the target nucleic acid sequence. U.S. Pat. No. 5,723,591 describes fluorescent hybridization probes that are fluorescence-quenched until hybridized to the target nucleic acid sequence, or until the probe is digested. Such techniques provide information about hybridization, and are of varying degrees of usefulness for the determination of single base variances in sequences. Some fluorescence techniques involve digestion of a nucleic acid hybrid in a 5' to 3' direction to release a fluorescent signal from proximity to a fluorescence quencher, for example, TaqMan.RTM. (Perkin Elmer; U.S. Pat. Nos. 5,691,146 and 5,876,930).
Real time PCR monitoring using fluorescence has been described in several manners. Firstly, the binding of double stranded DNA specific fluorescent dyes such as ethidium bromide allows for the monitoring of the accumulation of PCR product by correlation with increased fluorescence. A second detection method, polymerase mediated exonuclease cleavage utilises the 5' exonuclease activity of polymerases such as Taq. An oligonucleotide probe that is complementary to the PCR product, yet distinct from the PCR primer is labeled with a FRET pair such that the donor molecule is quenched by an acceptor molecule. During PCR amplification, the 5' exonuclease proceeds to digest the probe, seperating the FRET pair and leading to increased fluorescence. A variation on this technology uses a nucleic acid wherein the FRET pair is internally quenched, for example, by having a hairpin conformation. Upon hybridization to a sequence of interest, the FRET pair is separated and the donor molecule emits fluorescence. This technology can be used, for example for the analysis of SNPs.
An alternative technology is based on the use of two species of hybridization probes, each labeled with a member of a FRET pair. Upon hybridization of both probes to the target sequence in adequate proximity, a fluorescent signal is emitted. Again, this technology may be used for the detection of SNPs.
A major advantage of the use of such FRET based PCR technologies is that the reaction may be monitored in a closed tube reaction, suitable for use in high and medium throughput and reducing the probability of contamination.

### Methods on extracting and detecting DNA in bodily fluids

Methods for the detection of circulating DNA are described in a number of articles. In the majority of cases for separating the DNA from the biological sample scientists rely on a kit supplied by Qiagen, called QIAamp Blood Kit (Qiagen, Hilden, Germany):
For example in Jahr et al. (2001), Cancer Res 61: 1659-1655: "After having separated the plasma from blood cells by centrifugation at 3000g for 20 min the DNA from the blood plasma can be extracted using the QIAamp Blood Kit (Qiagen, Hilden, Germany) using the blood and body fluid protocol referring to Wong et al. (1999), Cancer Res 59: 71-73 and Lo et al. (1998) Am. J. Genet. 62: 768-775."
Wong et al. (1999), Cancer Res 59: 71-73: "Blood samples are centrifuged at 3000g and plasma and serum are carefully removed from the EDTA-containing and plain tubes, respectively, and transferred into plain polypropylene tubes. The buffy coat fraction from the EDTA-containing tubes was also collected to study the presence of circulating tumor cells in the peripheral blood. The samples were stored at -70C or -20C until further processing. DNA from plasma and serum samples was extracted using a QIAamp Blood Kit (Qiagen, Hilden, Germany) using the blood and body fluid protocol as recommended by the manufacturer (Chen et al. (1996). Microsatellite alterations in plasma DNA of small cell lung cancer patients. Nat Med 2: 1033-1035)."
Chen et al. : "Fresh frozen tissue was treated with SDS and proteinase K followed by phenol and chloroform extraction. Paraffin-embedded tissue was scraped from the slides and washed in xylol to remove paraffin. After the addition of one volume of ethanol, the mixture was centrifuged, and the pellet was digested with proteinase K and SDS, followed by phenol and chloroform extraction. Control Lymphoczte and plasma DNA were purified on Qiagen columns(Qiamp Blood Kit, Basel, Switzerland) according to the "blood and body fluid protocol". Plasma (1-3 ml) was passed on the same column. After purification, 1 ml of plasma yields an average of 39 ng of DNA.

The amounts of plasma DNA can be determined by competitive PCR according to the method of Diviacco et al. (1992) Gene 122: 313-320, using for example the Lamin B2 locus as a typical example for a single copy gene. The competitor molecule carrying a 20-bp insert was obtained directly from two amplification products by the overlap extension method (Diviacco et al. (1992) Gene 122: 313-320).
Quantitation of competitive templates can be obtained by OD260 measurement. A fixed amount of plasma DNA can be mixed with increasing amounts of the competitor template. For competitive PCR, two additional primers need to be designed. After PCR amplification and PAGE, two products are evidently corresponding to genomic and competitor templates. The ratios of the amplified products precisely reflect the initial concentration of genomic DNA versus that of the added competitor. Quantitation of competitor and genomic bands can be obtained by densitometric scanning of the ethidium bromide stained gel.
The results obtained by means of competitive PCR can be confirmed by quantitation with the control Kit DNA in the LightCycler System (Roche Diagnostics) using the LightCycler Control Kit DNA to amplify a 110 bp of the human Beta-globin gene. The amplicon can be detected by fluorescence using a specific pair of hybridization probes (LC-Red 640).

A similar approach was used by Lee et al. to quantify genomic DNA of serum and plasma samples DNA by using reagents from an HIV assay kit (HIV Monitor Assay, Roche Molecular Systems, Emeryville, CA). Immediately after thawing, plasma and serum samples were microcentrifuged at maximum speed (Microfuge II, Beckman Instruments) for 5 minutes to produce clean plasma or serum, free aggregates and non-specific precipitates. Plasma and/or serum (100ul) was removed and deposited into a 1.5 ml microcentrifuge tube containing 300 ul of working lysis reagent. The tube was then agitated vigorously for 3-5 seconds and incubated at room temperature for 10-15 minutes. After incubation, 400 ul of 100-percent isopropanol was added into each tube, which was then agitated for 3-5 seconds and microcentrifuged at 10000g (12000 rpm Microfuge II, Beckman Instruments)for 15 to 30 minutes at room temperatures. Supernatant was removed and 1 ml of 70 percent ethanol was added to each tube; these steps were followed by microcentrifugation at 10.000 g for 5 - 10 minutes at room temperature. The supernatant was removed and then the DNA pellets were left overnight at room temperature to evaporate any remaining ethanol. The pellet was resuspended in 100 ul of PCR solution A (100 mM KCl, 10 mM Tris, 2.5 mM MgCl_{2;} pH 8.3) and PCR solution B (10 mM Tris, 2.5 mM MgCl₂, 1% Tween-20, 1% Nonidet P-40; pH 8.3).
Purified DNA was amplified with HLA DQ-alpha primers or human γ-chromosome primers. Standard curves were prepared and for quantification included in each amplification (Lee et al. (2001) Transfusion 41: 276-282).
In the patent US 6156504 (Gocke et al.), which also relates to the detection of tumor-associated extracellular nuclec acid in plasma or serum fractions, an overview is given on several methods on how to extract and detect circulating DNA in blood and serum samples.

To determine the DNA concentration in a urine sample the samples need to be fresh, because human urine contains a nuclease activity (Botezatu et al. 2000). The fresh samples are centrifuged 10 min at 800 g and DNA is isolated from the supernatant as desccribed by Labarca and Paigen (Labarca and Paigen (1980) Anal Biochem 102: 344-352).

In summary, the state of the art is to develop more and more nucleic acid based assays in order to detect the presence or absence of tumor indicating protein or cDNA of tumor related genes, so called marker genes in blood or other bodily fluids. The detection of cancer specific alterations of genes involved in carcinogenesis, like oncogene mutations or deletions, tumor suppressor gene mutations or deletions, or microsatellite alterations will then allow a prediction of the patient to carry a tumor or not (for example patent WO 95/16792 or US 5,952,170 (Stroun et al.)). In an advanced stage the aim will be to produce a kit that allows the scientist to screen plenty of samples in little time with high accuracy. These kits will not only be of interest for an improved preventive medicine and early detection of cancer but also to monitor a tumors performance after therapy.
Also the detection of hypermethylation of certain genes, especially of certain promoter regions, has been recognized as an important indicator for the presence or absence of a tumor. To our knowledge so far all studies that dealt with methylation analysis looked at the methylation status of certain marker genes, only. These genes are known to play a role in the regulation of carcinogenesis or in other words are believed to determine the switching on and off of tumorigenesis. Most advanced is the knowledge about methylation and prostate cancer. Hence a method employing the methylation analysis of a certain marker gene (GSTP1) indicating prostate cancer using DNA from a bodily fluid has been patented (US 5,552,277). The determination of the methylation state of certain, yet to be identified indicator genes might even become a useful tool to predict the responsivness of a patient towards chemotherapy and radiotherapy (Hanna et al. (2001) Cancer Res 61: 2376-2380). On the other hand all those screening approaches are limited to certain cancer types. This is because they are all limited in that they look for certain marker genes, which are highly specific for a kind of cancer. The invention described in patent US 6,156,504 (Gocke et al.) also relates to the detection of extracellular nucleic acid in plasma or serum fractions but the patent only covers a method to detect mutated extracellular K-ras nucleic acid in blood. This is another example for the dependence of most assays on specific marker genes, in this case the oncogene K-ras. For a number of cancer types though these genes are not even known yet. Also, in an early screen, when there is no reason to assume that the patient suffers from a specific kind of cancer, a screen would require to test for every possible gene alteration known so far. This can be regarded as unfeasible.
On the other hand, cell proliferative diseases cause extracellular DNA levels in blood and in other bodily fluids to rise. To our knowledge the quantitation of extracellular DNA in humans has never been used to predict the risk of a patient to carry a cell proliferative disease like for example cancer. Some reports have been published where elevated levels of circulating DNA in blood of cancer patients are mentioned, but these were solely utilized as a source for easier accessible DNA in order to analyze its properties further (Jahr et al. 2001). It is also known that these DNA molecules origin from the tissue where the cells are dying, for whatever reason (as discussed above). Nevertheless, up to now there has been a lack of know-how in order to determine the DNAs origin and therefore it wasn't possible to link the general result of increased DNA levels in a bodily fluid like blood to the risk of a cell proliferative disease in a certain organ. This lack has been due to the inavailabity of tissue specific markers (Jahr et al. 2001), which would allow a determination of the DNAs origin. This is exactly the gap that our invention is able to close.
In short, the first result of an analysis of a bodily fluid from a screen would be an information about the circulating DNA level. In cases where this is elevated above normal (average from healthy people), which so far has not been seen as a significant risk factor on its own, would now lead to a further analysis in terms of methylation analysis. Without having to guess, which kind of cancer might be responsible for the emission of those DNA levels and without needing to employ assays on certain marker genes with our invention we will be able to reveal the DNA's origin. This is based on the discovery of tissue specific methylation patterns. With those we are able to interpret a certain methylation pattern as belonging to a certain tissue type.

It is therefore an object of the present invention to provide a method that enables a) a prediction that the patient is likely to suffer from a cell proliferative disease from determining the level of free floating, circulating DNA in his blood or other bodily fluid, and b) a prediction, which tissue is releasing the DNA and therefore is likely to carry the disease.

### BRIEF DESCRIPTION OF THE INVENTION

The present invention provides a method to determine the presence or absence of a medical condition like cancer or another cell proliferative disease. The method employs several steps starting with the retrieval of a person's sample in form of a tissue sample or a biological fluid like blood, serum, urine or other fluids as defined below and ending with the conclusive interpretation of data from a methylation pattern analysis of DNA captured from said sample, that gives information on the likelihood of the person suffering from a said disease. The method is based upon the quantification of free floating DNA in said biological fluid and the consequent determination of its methylation status. Determining the latter allows to decide where (as in from which organ) the potentially enhanced levels of DNA originate from. This allows to predict if the individual carries a cell proliferative disease like for example cancer in said organ. To validate this, the next step could be to employ for example a tailored test assay for disease indicating marker gene expression specific for said organ or tissue.
The idea to combine the quantitative analysis of DNA in a biological sample like blood with the consequent analysis of its methylation state in order to predict its origin is new and leads to new possibilities of screening large populations for very early signs of for example cancer even before clinical stages, when no other symptoms are noticeable yet. As early detection is the most important step in fighting a disease like cancer this method provides an important improvement towards a successful fight against these diseases. In addition the method can be employed to monitor the progression of a tumor after treatment and thereby allows to optimize the dosage of said treatment or adjusting to a different treatment in a patient specific individual manner.

### DETAILED DESCRIPTION OF THE INVENTION

'Bodily fluid' herein refers to a mixture of macromolecules obtained from an organism. This includes, but is not limited to, blood, blood plasma, blood serum, urine, sputum, ejaculate, semen, tears, sweat, saliva, lymph fluid, bronchial lavage, pleural effusion, peritoneal fluid, meningal fluid, amniotic fluid, glandular fluid, fine needle aspirates, nipple aspirate fluid, spinal fluid, conjunctival fluid, vaginal fluid, duodenal juice, pancreatic juice, bile and cerebrospinal fluid. This also includes experimentally separated fractions of all of the preceding. 'Bodily fluid' also includes solutions or mixtures containing homogenized solid material, such as feces.

A 'methyl-specific agent' herein refers to any chemical or enzyme interacting or reacting with nucleic acids in such a way that it differentiates between a methylated and a non-methylated nucleobase. By acting specifically on either the one or the other or by interacting with both in a different way it will be easier, by methods available today, to differentiate between these nucleobases than it has been before the interaction with said 'methyl-specific agents'. Examples for treatment with a 'methyl-specific agent' are the so called 'bisulfite treatment' or treatment with methylation sensitive restriction enzymes.

The term "bisulfite treatment" refers to the method commonly known to the person skilled in the art. Examples for the treatment can be found, for example, in several of the references cited herein.

In the context of the present invention, the term "hybridization" is to be understood as a bond of an oligonucleotide to a completely complementary sequence along the lines of the Watson-Crick base pairings in the sample DNA, forming a duplex structure.

One aspect of the present invention is to detect the presence or absence of and the monitoring of a disease in an individual, the disease being specified by showing increased levels of free, non cellular bound DNA in a bodily fluid as defined above. In a preferred embodiment of this invention this disease is a cell proliferative or neoplastic disease. In an especially preferred embodiment of this invention the disease is a type of cancer.
The present invention provides a method for the analysis of circulating nucleic acids. It discloses a means of distinguishing between healthy (or diseased) tissues from different sources in a human body. It is disclosed that typical methylation patterns of certain genes can be positively correlated with certain organs and tissues. This allows the identification of free floating DNA's origin, or in other words to determine the organic source of this circulating nucleic acids. This is done by in an assay that detects methylation at specific CpG sites by restriction enzyme analysis, or using a nucleic acid based method.

The invention hereby provides a means for the improved diagnosis, prognosis, staging and grading of cancer, at a molecular level, by employing the capacity to differentiate between sources of free floating DNA in bodily fluids. Also this new tool will help to discover the actual reason for the increase of nucleic acids in blood or serum for example.

Furthermore, the disclosed invention provides improvements over the state of the art in that current methods of methylation analysis are based on histological and cytological analyses that require a biopsy that provides a sufficient amount of tissue. The method according to the present invention can be used for classification of easily accessible samples like bodily fluids that do not require a biopsy.

The invention further provides a method for detecting the organic source of nucleic acids is characterized in that certain genes are contacted with a reagent or series of reagents capable of distinguishing between methylated and non methylated CpG dinucleotides within given target sequences.

The present invention further makes available a method for ascertaining genetic and/or epigenetic parameters of genomic DNA.

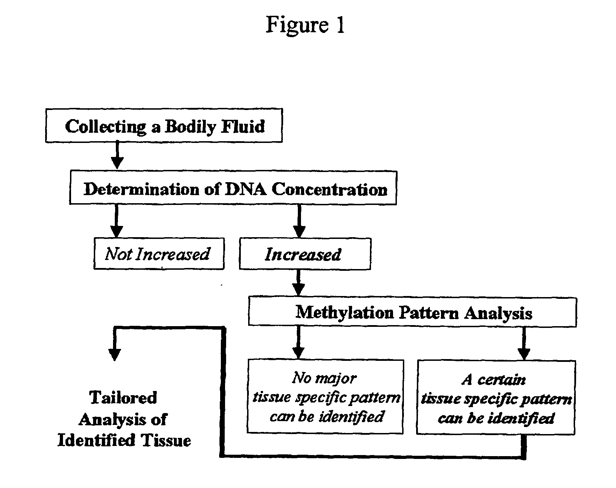

In a preferred embodiment, the method comprises the following steps, which are described with reference to **Figure 1** that shows a flow chart of the preferred method according to the present invention:

In the first step of the method, a sample is retrieved from a patient or individual in form of said bodily fluids (as defined above). The retrieval of the said sample can be done in any way known to a person skilled in the art. The detailed description can be found in relevant technical articles and text books that describe the state of the art. This includes but is not limited to ventricular puncture, also known as CSF collection, a procedure to obtain a specimen of cerebrospinal fluid (CSF); thoracentesis, referring to inserting a needle between the ribs into the chest cavity, using a local anesthetic to obtain the pleural effusion fluid; amniocentesis, referring to a procedure performed by inserting a hollow needle through the abdominal wall into the uterus and withdrawing a small amount of fluid from the sac surrounding the fetus; but also urine, sperm and sputum collection.

In a preferred embodiment the samples are obtained from any bodily fluids as mentioned in the definition above. In a further preferred embodiment the samples are obtained from whole blood, blood serum, urine, saliva or ejaculate from said individual.
In a second step, the extracellular nucleic acids from the bodily fluid are quantified. On this purpose the free floating nucleic acids may be extracted and/or separated from RNA if necessary. However the following steps are also enabled without doing any of the aforementioned treatment. Also, the DNA may be purified, or otherwise conditioned and prepared, before quantification. Purification may be done for example on Qiagen columns supplied in the Qiamp Blood Kit as described in Chen et al. (1996) (Nature medicine 2, 1033-1035). The quantitation may take place either immediately after retrieval of the sample or after an unspecified time of storage of said sample. In a preferred embodiment of the method the free floating DNA will be separated from the cell bound DNA via centrifugation either after the amount of total DNA in said sample (including the cell bound) has been determined or without determining the cell bound DNA at all.

Any process mentioned in step 2 may be done by means that are standard to one skilled in the art, these include the use of detergent lysates, sonification and vortexing with glass beads. In a preferred embodiment the sample is also conditioned by means of preservation, like heating or adding chemicals to deactivate or inhibit deoxyribonucleases or other nucleic acid degrading enzymes; storage at reduced (below room temperature) or not reduced temperatures; cooling; heating; the addition of detergents; filtering and/or centrifugation. For example the sample may be treated with proteinase K (from Boehringer Mannheim) and sodium dodecyl sulfate at 48°C overnight before separating out the DNA as described in Eisenberger et al (1999) (J Natl Cancer Inst 91: 2028-2032) for serum samples.
Also conditioning in this context comprises applying methods to concentrate the DNA in said sample. These methods can be either one or several of the methods mentioned in the description of prior art and may be any by means that are standard to one skilled in the art. Some of those are described in detail in Appendix E of the well known lab manual Sambrook, Fritsch and Maniatis (1989) Molecular Cloning - A Laboratory Manual (second edition): precipitation of DNA in microfuge tubes, precipitation of RNA with ethanol, concentrating nucleic acids by extraction with butanol (vol 2: E.12, E.15 and E.16 respectively).
In preferred embodiments conditioning can also mean any kind of chemical treatment, like adding an anti-coagulant, treatment with reducing agents, treatment with intercalating chemicals or chemicals that build covalent bonds with the DNA.
In a preferred embodiment the DNA may be cleaved prior to the chemical treatment, this may be by any means standard in the state of the art, in particular with restriction endonucleases.
The quantitation of the free floating DNA may also be done by any means that are standard to one skilled in the art. Commonly used techniques are based on spectrophotometric and/or fluorometric analyses, for example: the concentration of a dilute sample of plasmid DNA purified by two passes through an ethidium bromide - cesium chloride (EtBr-CsCI) centrifugation gradient can either be determined on an for example LKB Biochrom Ultrospec II spectrophotometer for absorbance at wavelengths of 260 nm and 280 nm, or it can be tested for emission of 460 nm on the Hoefer TKO 100 mini-fluorometer in the presence of bisbenzimidizole, a fluorescent dye known as Hoechst H 33258 (manufactured by American Hoechst Corporation), that has an excitation maximum at 356 nm and an emission maximum of 458 when bound to DNA (Labarca and Paigen (1980) Anal. Biochem. 102, 344-352). The spectrophotometer detects absorbance due to RNA as well as DNA, while the Hoechst dye used in the fluorometer interacts specifically with adenosine and thymidine residues of DNA. In a preferred embodiment the Invitrogen's nucleic acid quantitation DNA Dipstick^{[™]} kit is used, which is claimed to be sensitive enough to detect as little as 0.1 ng/ul of nucleic acid. Unfortunately, the method cannot be used with samples containing more than 10 ng/ul of nucleic acids (Trends in Biochemical Sciences 19, 46-47).
The total amount of free floating DNA may be measured for example by intercalating fluorescent dyes or other dyes changing their fluorescence properties when binding to DNA, and also by hybridization to DNA specific probes including, but not limited to oligonucleotides or PNA (peptide nucleic acid) oligomers, real time PCR assays or other real time amplification procedures, UV-Vis absorbance or in general amplification procedures with subsequent determination of the amount of product formed.

In a third step, the methylation pattern of the free floating DNA is determined in order to discover where the majority of DNA origins from.
In order to do so, the nucleic acid sample is first treated with a 'methyl-specific agent' like, but not limited to, bisulfite or with for example methylation sensitive restriction enzymes. In a preferred embodiment the extracellullar nucleic acids are chemically treated in such a manner that cytosine bases which are unmethylated at the 5'-position are converted to uracil, thymine, or another base which is dissimilar to cytosine in terms of hybridization behavior. This will be understood as treatment with a 'methyl-specific agent'. Said chemical conversion may take place in any format standard in the art. This includes but is not limited to modification within agarose gel or in denaturing solvents. The nucleic acid may be, but doesn't have to be, concentrated and/or otherwise conditioned before the said nucleic acid sample is treated with said agent. In this third step of the method, it is preferred that the above described treatment of extracellular nucleic acids is carried out with bisulfite (sulfite, disulfite) and subsequent alkaline hydrolysis, which results in a conversion of non-methylated cytosine nucleobases to uracil or to another base which is dissimilar to cytosine in terms of base pairing behavior.
The double stranded DNA is preferentially denatured. This may take the form of a heat denaturation carried out at variable temperatures. The denaturation temperature is generally depending on the buffer but for high molecular weight DNA it can be as high as 90°C. However, the analysis may be upon smaller fragments which do not require such high temperatures. In addition as the reaction proceeds and the cytosine residues are converted to uracil the complementarity between the strands decreases. Therefore, a cyclic reaction protocol may consist of variable denaturation temperatures.
The bisulfite conversion then consists of two important steps, the sulfonation of the cytosine and the subsequent deamination. The equilibra of the reaction are on the correct side at two different temperatures for each stage of the reaction. The temperatures and length at which each stage is carried out may be varied according to the specific requirement of the situation. However, a preferred variant of the method comprises a change of temperature from 4°C (10 minutes) to 50°C (20 minutes). This form of bisulfite treatment is state of the art with reference to WO 99/28498.

Said chemical conversion may take place in any format standard in the art. This includes but is not limited to modification within agarose gel, in denaturing solvents or within capillaries.

In preferred embodiments bisulfite conversion within agarose gel will be done as described by Olek et al, Nucl. Acids. Res. 1996, 24, 5064-5066. The DNA fragment is embedded in agarose gel and the conversion of cytosine to uracil takes place with hydrogensulfite and a radical scavenger. The DNA may then be amplified without need for further purification steps.

Furthermore, the specific modifications in said nucleic acids caused by said treatment are detected by use of the standard methods as described below.
For a number of healthy organs and tissues certain CpG sites have been identified and are disclosed in this invention that are specifically methylated. From the pool of different nucleic acids circulating in the bodily fluid, these sites are tested for their methylation status. This is done in a way as described below: Fragments of the chemically pretreated DNA are amplified, using sets of primer oligonucleotides and a, preferably heat-stable polymerase. Because of statistical and practical considerations, preferably more than ten different fragments having a length of 100 - 2000 base pairs are amplified. The amplification of several DNA segments can be carried out simultaneously in one and the same reaction vessel. Usually, the amplification is carried out by means of a polymerase chain reaction (PCR).
The method may also be enabled by the use of alternative primers, the design of such primers is obvious to one skilled in the art. These should include at least two oligonucleotides whose sequences are each reverse complementary or identical to an at least 18 base-pair long segment of the base sequences. Said primer oligonucleotides are preferably characterized in that they do not contain any CpG dinucleotides. In a particularly preferred embodiment of the method, the sequence of said primer oligonucleotides are designed so as to selectively anneal to and amplify, only the tissue specific DNA of interest, thereby minimizing the amplification of background or non relevant DNA. In the context of the present invention, background DNA is taken to mean genomic DNA which does not have a relevant tissue specific methylation pattern, in this case, the relevant tissue being one tissue out of the plenty we have found marker genes for, both healthy and diseased.

According to the present invention, it is preferred that at least one primer oligonucleotide is bound to a solid phase during amplification. The different oligonucleotide sequences can be arranged on a plane solid phase in the form of a rectangular or hexagonal lattice, the solid phase surface preferably being composed of silicon, glass, polystyrene, aluminum, steel, iron, copper, nickel, silver, or gold, it being possible for other materials such as nitrocellulose or plastics to be used as well.

The fragments obtained by means of the amplification can carry a directly or indirectly detectable label. Preferred are labels in the form of fluorescence labels, radionuclides, or detachable molecule fragments having a typical mass which can be detected in a mass spectrometer, it being preferred that the fragments that are produced have a single positive or negative net charge for better detectability in the mass spectrometer. The detection may be carried out and visualized by means of matrix assisted laser desorption/ionization mass spectrometry (MALDI) or using electron spray mass spectrometry (ESI).
The amplificates obtained are subsequently hybridized to an array or a set of oligonucleotides and/or PNA (peptide nucleic acid) probes. In this context, the hybridization takes place in the manner described in the following. The set of probes used during the hybridization is preferably composed of at least 10 oligonucleotides or PNA-oligomers. In the process, the amplificates serve as probes, which hybridize to oligonucleotides previously bonded to a solid phase. The non-hybridized fragments are subsequently removed. Said oligonucleotides contain at least one base sequence having a length of 10 nucleotides which is reverse complementary or identical to a segment of the base sequences specified in the appendix, the segment containing at least one CpG dinucleotide. The cytosine of the CpG dinucleotide is the 5^{th} to 9^{th} nucleotide from the 5'-end of the 10-mer. One oligonucleotide exists for each CpG dinucleotide. Said PNA-oligomers contain at least one base sequence having a length of 9 nucleotides which is reverse complementary or identical to a segment of the base sequences specified in the appendix, the segment containing at least one CpG dinucleotide. The cytosine of the CpG dinucleotide is the 4^{th} to 6^{th} nucleotide seen from the 5'-end of the 9-mer. Preferably one oligonucleotide exists for each CpG dinucleotide.

In the next step, the non-hybridized amplificates are removed.

In the final step of this procedure, the hybridized amplificates are detected. In this context, it is preferred that labels attached to the amplificates are identifiable at each position of the solid phase at which an oligonucleotide sequence is located.

According to the present invention, it is preferred that the labels of the amplificates are fluorescence labels, radionuclides, or detachable molecule fragments having a typical mass which can be detected in a mass spectrometer. The mass spectrometer is preferred for the detection of the amplificates, fragments of the amplificates or of probes which are complementary to the amplificates, it being possible for the detection to be carried out and visualized by means of matrix assisted laser desorption/ionization mass spectrometry (MALDI) or using electron spray mass spectrometry (ESI). The produced fragments may have a single positive or negative net charge for better detectability in the mass spectrometer.

The methylation patterns found in the tested sample will be identified as belonging to a certain tissue or organ. This is done by checking the methylation pattern of certain organ specific marker genes or by comparing the methylation pattern of a broader range of several genes to the pattern of those same genes when extracted from different tissues and organs. An individual dataset is compared to data received in previous studies or a dataset obtained in a parallel experiment on a control fluid.
This analysis will reveal the predominance of a certain source of DNA. Thereby the amount of free floating DNA that originates from this tissue, specified in that it is showing a certain specific methylation status, relative to the total amount of free floating DNA is determined.

In a fourth step, the presence or absence of a medical condition in said organ is determined by comparing the individual's test result, regarding the ratio of a single sourced DNA to the amount of total DNA, with the dataset that was built up in house in previous studies. This is based on the ratio of the fraction of free floating DNA that originates from a specific tissue or organ and the total amount of free floating DNA. Based on these results it is posssible to identify patients with abnormal amounts of DNA of a certain organ or tissue, as in increased by more than 10% above a value defined as "normal", in their bodily fluids. In a preferred embodiment it is possible to positively identify patients with free floating DNA levels increased by at least but not limited to 20% above a value defined as normal. In a further preferred embodiment it is possible to identify patients with an increased level of free floating DNA, specified in increased by at least but not limited to 40% above normal.
Furthermore and most importantly the said analysis will not only tell that the patients DNA level are increased but also reveal the possible cause of it, as in specifying where this extracellular DNA comes from. This will give the doctor or clinician involved a valueable tool to identify a disease in its very early days, even before noticable symptoms might have occurred.

The invention provides a method as described above characterized in that the said methylation pattern is found to be specific for said organ or tissue with regards to other organs or tissues. In a preferred embodiment the method is characterized in that the said methylation pattern is found to be specific for said organ or tissue with regards to methylation patterns that can be found in DNA from other organs or tissues, specified by the fact that it is not found in other organs or tissues which are involved in the medical condition of interest and thereby independent of the medical condition the patient might be diagnosed with.

In a further preferred embodiment the method is characterized in that the said methylation pattern is found to be specific for said organ or tissue with regards to other organs or tissues when the medical condition the patient is diagnosed with is a tumor or another cell proliferative disease.

The invention provides a method for determining the fraction of free floating DNA in a bodily fluid that originates from an organ or tissue of interest, characterized in that the following steps are carried out: First, retrieving a bodily fluid sample from said individual as described above; second, detecting the amount of total free floating DNA in said sample as described above; third determining the amount of free floating DNA that originates from a specific tissue or organ by determining the amount of free floating DNA that has a methylation pattern characteristic for a specific tissue which was determined previously; fourth, determining the fraction of total free floating DNA which originates from said tissue or organ; fifth, concluding, if there is an abnormal level of total free floating DNA, whether this DNA originates from said tissue or organ and sixth, concluding whether a medical condition associated with said tissue or organ is present.

In a further embodiment the invention provides a method for determining the fraction of free floating DNA in a bodily fluid that originates from an organ or tissue of interest, characterized in that the following steps are carried out: First, retrieving a bodily fluid sample from said individual; second, conditioning said sample to prepare the binding of free floating DNA to a surface; third, detecting the amount of total free floating DNA by measuring the amount of DNA bound to said surface; fourth, subjecting said surface with the immobilized DNA to a chemical and/or enzymatic treatment that converts all unmethylated cytosines in the DNA into uracil but leaving in position 5 methylated cytosines unchanged as described above; fifth, amplifying the treated DNA; sixth, analyzing several positions in said treated DNA and determining the amount of DNA that has a tissue specific DNA methylation pattern which was determined previously; seventh, determining the fraction of total free floating DNA that originates from said tissue or organ.

In a further embodiment the method includes the following steps: If there is an abnormal level of total free floating DNA it is concluded whether this DNA originates from said tissue or organ and whether a medical condition associated with said tissue or organ is present.

The present invention is also directed to a method for diagnosing a disease or medical condition that comprises any of the methods that are disclosed in this invention.

In addition, a means to produce a device to determine the total amount of free floating DNA in a bodily fluid, comprising a surface to bind DNA floating in a sample volume of bodily fluid and a means for detecting the amount of DNA bound to this solid surface is disclosed. The device is further characterized in that it comprises a chamber to host the surface and reagents to chemically or enzymatically modify the DNA bound to said surface and a means to control and adjust the temperature in this chamber.
Said surface may be the same as described and used in the DNA DipStick™ kit (supplied by Invitrogen) or of other means enabling DNA to selectively bind to a material applicated to some unspecified kind of carrier, which might be either mobile or fixed. The binding may for example be based on unspecific hybridization of nucleic acids. The quantification of DNA bound to said surface may be carried out by any means standard to anyone skilled in the art or for example following instructions given in the DNA DipStick™ Kit. Furthermore a means how to produce a chamber or similar kind of closed environment to host said surface together with the required reagents and / or enzymes to modify the DNA bound to said solid surface is disclosed.
The means to control and adjust the temperature in this chamber may be done by means that are standard to anyone skilled in the art, for example by fixing an electronic thermometer or any device able to read the temperature and connect it to a chip programmed to react in a certain way by switching on a cooling or heating unit.
However, a kit can also contain only parts of the aforementioned components and may not include the device. It may be composed, for example, of a bisulfite-containing reagent, a set of primer oligonucleotides containing at least two oligonucleotides whose sequences in each case correspond or are complementary to a 18 base long segment of a specific base sequence, oligonucleotides and/or PNA (peptide nucleic acid)-oligomers as well as instructions for carrying out and evaluating the described method.

## Claims

1. A method for detecting the presence or absence of a diseased condition in a tissue or organ of an individual, comprising the following steps:
a) determining the amount of total free floating DNA in a bodily fluid sample obtained from said individual;
b) determining the amount of free floating DNA that originates from said tissue or organ in said sample;
c) determining the presence or absence of a diseased condition based on the total amount of free floating DNA and the fraction of free floating DNA that originates from said tissue or organ.

2. A method according to claim 1, **characterized in that** the sample is conditioned before the amount of free floating DNA is detected.

3. A method according to claim 2, **characterized in that** the sample is conditioned by means of centrifugation, filtering, heating, cooling, concentration or chemical treatment.

4. A method according to one of the preceding claims, **characterized in that** the amount of DNA originating from a certain organ or tissue is determined by analyzing a DNA methylation pattern that is characteristic for said organ or tissue.

5. A method according to claim 4, **characterized in that** said methylation pattern is found in said organ or tissue and not in other organs or tissues involved in the diseased condition of interest.

6. A method according to one of the preceding claims, **characterized in that** the diseased condition is a tumor or another cell proliferative disease.

7. A method according to one of the preceding claims, **characterized in that** the samples are obtained from bodily fluids like whole blood, blood plasma, blood serum, urine, sputum, ejaculate, semen, tears, sweat, saliva, lymph fluid, bronchial lavage, pleural effusion, peritoneal fluid, meningal fluid, amniotic fluid, glandular fluid, fine needle aspirates, nipple aspirate fluid, spinal fluid, conjunctival fluid, vaginal fluid, duodenal juice, pancreatic juice, bile and cerebrospinal fluid from said individual.

8. A method according to one of the preceding claims, **characterized in that** the methylation pattern is determined by subjecting the DNA to a chemical or enzymatic treatment that converts all unmethylated cytosines in the DNA into uracil but leaving position 5-methylated cytosines unchanged.

9. A method according to claim 1, wherein step b) comprises
I) determining the amount of free floating DNA that originates from said tissue or organ by determining the amount of free floating DNA that has a tissue or organ characteristic DNA methylation pattern;
II) determining the fraction of total free floating DNA that originates from said tissue or organ; and wherein step c) comprises
III) concluding, whether there is an abnormal level of free floating DNA that originates from said tissue or organ and
IV) concluding whether a diseased condition associated with said tissue or organ is present.

10. A method according to claim 1, comprising the following steps
a) conditioning a bodily fluid sample obtained from an individual to prepare the binding of free floating DNA to a surface;
b) binding at least part of said DNA to said surface;
c) detecting the amount of total free floating DNA by measuring the amount of DNA bound to said surface;
d) subjecting said surface comprising said bound DNA to a chemical and/or enzymatic treatment that converts all unmethylated cytosines in the DNA into uracil but leaving position-5 methylated cytosines unchanged;
e) amplifying the treated DNA; and
f) analyzing several positions in said treated DNA and determining the amount of DNA that has a tissue or organ-characteristic DNA methylation pattern; and
g) determining the fraction of total free floating DNA that originates from said tissue or organ.

11. The method of claim 10, **characterized in that** the following additional steps are carried out:
h) concluding, whether this DNA originates from said tissue or organ, if there is an abnormal level of total free floating DNA; and
i) concluding, whether a diseased condition associated with said tissue or organ is present.

12. A method according to one of the preceding claims, **characterized in that** the total amount of free floating DNA is measured by intercalating fluorescent dyes or other dyes changing their fluorescence properties when binding to DNA, hybridization to DNA specific probes including, but not limited to oligonucleotides or PNA oligomers, real time PCR assays or other real time amplification procedures, UV-Vis absorbance or in general amplification procedures with subsequent determination of the amount of product formed.

## Patentansprüche

1. Verfahren zum Nachweis der Anwesenheit oder Abwesenheit eines Erkrankungszustandes in einem Gewebe oder Organ eines Individuums, umfassend die folgenden Schritte:
a) Bestimmen der Menge von gesamter frei-flottierender DNA in einer Körperflüssigkeitsprobe, die von dem Individuum erhalten wurde;
b) Bestimmen der Menge von frei-flottierender DNA in der Probe, die von dem Gewebe oder Organ abstammt;
c) Bestimmen der Anwesenheit oder Abwesenheit eines Erkrankungszustandes, basierend auf der Gesamtmenge von frei-flottierender DNA und der Fraktion von frei-flottierender DNA, die von dem Gewebe oder Organ abstammt.

2. Verfahren nach Anspruch 1, **dadurch gekennzeichnet, daß** die Probe aufbereitet wird, bevor die Menge von frei-flottierender DNA nachgewiesen wird.

3. Verfahren nach Anspruch 2, **dadurch gekennzeichnet, daß** die Probe mittels Zentrifugation, Filtration, Erhitzen, Abkühlen, Konzentrieren oder chemischer Behandlung aufbereitet wird.

4. Verfahren nach einem der voranstehenden Ansprüche, **dadurch gekennzeichnet, daß** die Menge von DNA, die von einem bestimmten Organ oder Gewebe abstammt, durch Analysieren eines DNA-Methylierungsmusters bestimmt wird, das für das Organ oder Gewebe charakteristisch ist.

5. Verfahren nach Anspruch 4, **dadurch gekennzeichnet, daß** das Methylierungsmuster in dem Organ oder Gewebe gefunden wird und nicht in anderen Organen oder Geweben, die an dem Erkrankungszustand von Interesse beteiligt sind.

6. Verfahren nach einem der voranstehenden Ansprüche, **dadurch gekennzeichnet, daß** der Erkrankungszustand ein Tumor oder eine andere proliferative Erkrankung ist.

7. Verfahren nach einem der voranstehenden Ansprüche, **dadurch gekennzeichnet, daß** die Proben von Körperflüssigkeiten erhalten werden, wie etwa Gesamtblut, Blutplasma, Blutserum, Urin, Sputum, Ejakulat, Samen, Tränen, Schweiß, Speichel, Lymphflüssigkeit, bronchialer Lavage, pleuraler Effusion, peritonealer Flüssigkeit, meningaler Flüssigkeit, amniotischer Flüssigkeit, glandulärer Flüssigkeit, feiner Nadelaspirate, Brustwarzenaustrittsflüssigkeit, Spinalflüssigkeit, Konjunktivalflüssigkeit, Vaginalflüssigkeit, duodenaler Säfte, pankreatischer Säfte, Gallenflüssigkeit und zerbospinaler Flüssigkeit von dem Individuum.

8. Verfahren nach einem der voranstehenden Ansprüche, **dadurch gekennzeichnet, daß** das Methylierungsmuster durch Unterziehen der DNA einer chemischen oder enzymatischen Behandlung bestimmt wird, die alle nicht methylierten Cytosine in der DNA in Uracil überführt, jedoch Position 5-methylierte Cytosine unverändert läßt.

9. Verfahren nach Anspruch 1, wobei Schritt b) umfaßt:
I) Bestimmen der Menge von frei-flottierender DNA, die von dem Gewebe oder Organ abstammt, durch Bestimmen der Menge von frei-flottierender DNA, die ein Gewebe- oder Organ-charakteristisches DNA-Methylierungsmuster aufweist;
II) Bestimmen der Fraktion von gesamter frei-flottierender DNA, die von dem Gewebe oder Organ abstammt;
und worin Schritt c) umfaßt
III) Schlußfolgern, ob ein abnormaler Spiegel von frei-flottierender DNA vorhanden ist, die von dem Gewebe oder Organ abstammt; und
IV) Schlußfolgern, ob ein mit dem Gewebe oder Organ assoziierter Erkrankungszustand vorhanden ist.

10. Verfahren nach Anspruch 1, umfassend die folgenden Schritte
a) Aufbereiten einer Körperflüssigkeitsprobe, die von einem Individuum erhalten wurde, um die Bindung von frei-flottierender DNA an eine Oberfläche vorzubereiten;
b) Binden von mindestens einem Teil der DNA an die Oberfläche;
c) Nachweisen der Menge von gesamter frei-flottierender DNA durch Messen der Menge von DNA, die an die Oberfläche gebunden wurde;
d) Unterziehen der Oberfläche, die die gebundene DNA umfaßt, einer chemischen und/oder enzymatischen Behandlung, die alle nicht methylierten Cytosine in der DNA in Uracil überführt, wobei jedoch Position-5-methylierte Cytosine unverändert bleiben;
e) Amplifizieren der behandelten DNA; und
f) Analysieren von mehreren Positionen in der behandelten DNA und Bestimmen der Menge von DNA, die ein Gewebe- oder Organ-charakteristisches DNA-Methylierungsmuster aufweist; und
g) Bestimmen der Menge von gesamter frei-flottierender DNA, die von dem Gewebe oder Organ abstammt.

11. Verfahren nach Anspruch 10, **dadurch gekennzeichnet, daß** die folgenden zusätzlichen Schritte durchgeführt werden:
h) Schlußfolgern, ob diese DNA von dem Gewebe oder Organ abstimmt, falls ein abnormaler Spiegel von gesamter frei-flottierender DNA vorhanden ist; und
i) Schlußfolgern, ob ein mit dem Gewebe oder Organ assoziierter Erkrankungszustand vorhanden ist.

12. Verfahren nach einem der voranstehenden Ansprüche, **dadurch gekennzeichnet, daß** die gesamte Menge von frei-flottierender DNA durch interkalierende Fluoreszenzfarbstoffe oder andere Farbstoffe gemessen wird, die ihre Fluoreszenzeigenschaften ändern, wenn sie an DNA binden, Hybridisierung an DNA-spezifische Sonden einschließlich, jedoch nicht begrenzt auf, Oligonukleotide oder PNA-Oligomere, Echt-Zeit-PCR-Tests oder andere Echt-Zeit-Amplifikationsverfahren, UV-Vis-Absorption oder im allgemeinen Amplifikationsverfahren mit anschließender Bestimmung der Menge von gebildeten Produkt.

## Revendications

1. Procédé pour détecter la présence ou l'absence d'un état pathologique dans un tissu ou un organe d'un individu, comprenant les étapes suivantes :
a) détermination de la quantité d'ADN libre total flottant dans un échantillon de liquide organique obtenu dudit individu ;
b) détermination de la quantité d'ADN libre flottant qui provient dudit tissu ou dudit organe dans ledit échantillon ;
c) détermination de la présence ou de l'absence d'un état pathologique sur la base de la quantité totale d'ADN libre flottant et de la fraction d'ADN libre flottant qui provient dudit tissu ou organe.

2. Procédé selon la revendication 1, **caractérisé en ce que** l'échantillon est conditionné avant la détection de la quantité d'ADN libre flottant.

3. Procédé selon la revendication 2, **caractérisé en ce que** l'échantillon est conditionné au moyen d'une centrifugation, d'une filtration, d'un chauffage, d'un refroidissement, d'une concentration ou d'un traitement chimique.

4. Procédé selon l'une quelconque des revendications précédentes, **caractérisé en ce que** la quantité d'ADN provenant d'un certain organe ou tissu est déterminée par analyse d'un modèle de méthylation de l'ADN qui est caractéristique dudit organe ou tissu.

5. Procédé selon la revendication 4, **caractérisé en ce que** ledit modèle de méthylation est trouvé dans ledit organe ou tissu et non dans d'autres organes ou tissus impliqués dans l'état pathologique d'intérêt.

6. Procédé selon l'une quelconque des revendications précédentes, **caractérisé en ce que** l'état pathologique est une tumeur ou une autre maladie de prolifération cellulaire.

7. Procédé selon l'une quelconque des revendications précédentes, **caractérisé en ce que** les échantillons sont obtenus des liquides organiques tels que le sang total, le plasma sanguin, le sérum sanguin, l'urine, les crachats, les éjaculats, le sperme, les larmes, la sueur, la salive, le liquide lymphatique, un lavage bronchique, un épanchement pleural, le liquide péritonéal, le liquide méningé, le liquide amniotique, le liquide glandulaire, le liquide aspiré par aiguille fine, le liquide aspiré par mamelon, le liquide spinal, le liquide conjonctival, le liquide vaginal, suc duodénal, un suc pancréatique, de la bile et un liquide céphalo-rachidien dudit individu.

8. Procédé selon l'une quelconque des revendications précédentes, **caractérisé en ce que** le modèle de méthylation est déterminé en soumettant l'ADN à un traitement chimique ou enzymatique qui transforme toutes les cytosines non méthylées dans l'ADN en uracile mais laisse les cytosines méthylées en position 5 inchangées.

9. Procédé selon la revendication 1, dans lequel l'étape b) comprend :
I) la détermination de la quantité d'ADN libre flottant qui provient dudit tissu ou organe en déterminant la quantité d'ADN libre flottant qui a un modèle de méthylation de l'ADN caractéristique d'un tissu ou d'un organe ;
II) détermination de la fraction d'ADN libre total qui provient dudit tissu ou organe ;
et dans lequel l'étape c) comprend
III) la conclusion selon laquelle il y a un niveau anormal d'ADN libre qui provient dudit tissu ou organe ou non ; et
IV) la conclusion selon laquelle un état pathologique associé audit tissu ou organe est présent ou non.

10. Procédé selon la revendication 1, comprenant les étapes suivantes :
a) conditionnement d'un échantillon de liquide organique obtenu d'un individu pour préparer la liaison de l'ADN libre flottant à une surface ;
b) liaison d'au moins une partie dudit ADN à ladite surface ;
c) détection de la quantité d'ADN libre total en mesurant la quantité d'ADN lié à ladite surface ;
d) soumission de ladite surface comprenant ledit ADN lié à un traitement chimique et/ou enzymatique qui transforme toutes les cytosines non méthylées dans l'ADN en uracile mais qui laisse les cytosines méthylées en position 5 inchangées ;
e) amplification de l'ADN traité ; et
f) analyse de plusieurs positions dans ledit ADN traité et détermination de la quantité d'ADN qui a un modèle de méthylation de l'ADN caractéristique d'un tissu ou d'un organe ; et
g) détermination de la fraction d'ADN libre total qui provient dudit tissu ou organe.

11. Procédé selon la revendication 10, **caractérisé en ce que** les étapes supplémentaires suivantes sont réalisées :
h) conclusion selon laquelle cet ADN provient dudit tissu ou organe ou non, et s'il existe un niveau anormal d'ADN libre total flottant ou non ; et
i) conclusion selon laquelle un état pathologique associé audit tissu ou organe est présent ou non.

12. Procédé selon l'une quelconque des revendications précédentes, **caractérisé en ce que** la quantité totale d'ADN libre flottant est mesurée en intercalant des colorants fluorescents ou d'autres colorants dont les propriétés fluorescentes changent lors d'une liaison à l'ADN, hybridation à des sondes spécifiques de l'ADN comprenant, mais sans s'y limiter, des oligonucléotides ou des oligomères PNA, des dosages par PCR en temps réel ou d'autres protocoles d'amplification en temps réel, une absorbance UV-lumière visible ou des protocoles d'amplification en général avec une détermination ultérieure de la quantité de produit formée.
